# EUROPEAN PATENT APPLICATION

(11) **EP 3 087 914 A1**
(43) Date of publication of application: **02.11.2016**
(21) Application number: 16167753.9
(22) Date of filing: 29.04.2016
(51) Int. Cl.: A61B 5/00, A61B 5/02, A61B 5/021, A61B 5/0295, A61B 5/053, A61B 5/11, G01G 19/50

(54) **WEIGHING SCALE WITH EXTENDED FUNCTIONS**

(30) Priority: 30.04.2015 US 201514701155
(71) Applicant: Withings, 92130 Issy les Moulineaux (FR)
(72) Inventor: BUARD, Nadine, 92190 Meudon (FR); CAMPO, David, 75015 Paris (FR); YU, Roger, 93360 Neuilly-Plaisance (FR); TECHER, Frédéric, 77120 Coulommiers (FR); BARROCHIN, Pierre, 92120 Montrouge (FR); CARREEL, Eric, 92190 Meudon (FR); PALLAS ARENY, Ramon, 08028 Barcelona (ES); CASAS PIEDRAFITA, Jaime Oscar, 08950 Esplugues de Llobregat (ES)
(74) Representative: Cabinet Plasseraud

(57) **Abstract**

**Method** to determine the **arterial stiffness** of an individual user (U) standing on a personal electronic scale (1) having a top surface with at least **three conductive pads** (11-13) on **one** of the left or right side of the scale,
**/a/-** acquiring weight variations, and extracting therefrom a ballistocardiogram (21) of the user's **heart beat,**
**/b/-** acquiring impedance plethysmography signals across one of the foot of the user, and extracting therefrom a **blood pulse signal** (22) at the foot,
**/c/-**calculating a **time delay** (DT) between the **heart beat** and the **blood pulse signal** arriving at the foot,
**/d/** deducing therefrom a value of the **arterial stiffness** of the user

## Description

### FIELD OF THE INVENTION

The present invention relates to weighing scale with extended functions, especially scales that provide, additionally to weight, information about some cardiovascular parameters.

### BACKGROUND OF THE DISCLOSURE

In the known art, it is known from US8639226 [to Withings] to measure a body fat percentage of a user standing barefoot on a scale. Besides, it is known from WO2014106716 [to Withings] to determine the heart rate of a user standing on a scale, by weight variations and foot-to-foot impedance analysis.

There is a need to provide from such a scale more information about health and physiological parameters of the user. There have been attempts to provide information about cardiovascular system like a rating of the arterial stiffness, like from example in document US2013/310700 [to Stanford]. However, photoplethysmograpy is a technique which suffers shortcomings when applied to a sole of a foot. Indeed the skin is substantially thicker at this place than at other locations where photoplethysmograpy is currently used. Also, there are few arteries located close to the skin of the foot sole.

Therefore, there is still a need to bring new solutions to provide information about cardiovascular system like a rating of the arterial stiffness.

### SUMMARY OF THE DISCLOSURE

According to a **first** aspect of the present disclosure, it is disclosed a method to determine the arterial stiffness of an individual user (U) standing on a personal electronic scale (1) having a top surface with at least three conductive pads on one of the left or right side of the scale, the method comprising:
**/a/-** acquiring weight variations, and extracting therefrom a ballistocardiogram (21) of the user's heart beat,
**/b/-** acquiring impedance plethysmography signals across one of the feet of the user, and extracting therefrom a blood pulse signal (22) at the foot, said blood pulse signal being a signal representative of an increase of a volume of blood in the foot upon arrival of the pressure pulse,
**/c/**-calculating a time delay (DT) between the heart beat and the blood pulse signal arriving at the foot,
**/d/** deducing therefrom a value of the arterial stiffness of the user;
whereby a reliable, user-friendly and non-invasive method is provided to assess the arterial stiffness of a user, which is a useful information regarding the health state of the user.

In various embodiments of the disclosure, one may possibly have recourse in addition to one and/or other of the following arrangements.
Advantageously, at step **/b/** the method preferably comprises:
**/b1/-** injecting into the foot a current between a first pad and a second pad,
**/b2/-** measuring resulting voltage across a third pad and a fourth pad,
whereby the impedance plethysmography measurement is made more reliable, by decoupling the current injection and the voltage measurement.
Advantageously the injected current is a sine alternating current, and the measured voltage is demodulated to obtain the impedance plethysmogram.
Advantageously, at step **/d/,** the deduction of a value of the arterial stiffness can be made according to a profile of the user, said profile including the height, the age, the gender and the blood pressure of the user. The arterial stiffness information is thereby rendered more specific and relevant to the particular user.
Advantageously, at step **/d/,** the value of the arterial stiffness can be compared to a previous value of the arterial stiffness of the same user. According to such a time-differential mode, a relevant change over time in the user's cardiovascular system can be found.
Advantageously, at step /c/, the time delay (DT) can be calculated as an average over at least 3 heart beats. Thereby the time delay calculation and therefore the arterial stiffness information can be calculated more reliably.
According to another aspect, there can be provided at least three conductive pads on the left side of the scale and at least three conductive pads on the right side of the scale, and steps /a/ to **/d/** can be performed for the left foot and for the right foot, subsequently or in parallel, and a resulting average value is outputted together with a user notice if the left and right values differ of more than a predetermined value.

According to another aspect of the disclosure, there is also proposed an electronic scale having at least three conductive pads on one of the left or right side of a top surface of the scale, wherein a first and a second pads are used to circulate a current into one foot of a user, (from the front to the rear portions of the foot sole or conversely), and wherein a third and a fourth pads are used to measure the resulting voltage current across the same foot.
The first pad and the third pad can be arranged on the front portion of the top surface of the scale, and the second pad and the fourth pad are arranged on the rear portion of the top surface the scale. Thereby, both current and voltage are handled over a significant part of the foot and the impedance is measured over a significant length of the foot thereby increasing accuracy.

Preferably, the third and fourth pads are interposed between first pad and second pad. Preferably, the front pads (first and third) are arranged symmetrically to the rear pads (second and fourth) with regard to the medial transversal axis Y.

The fourth pad and the second pad can be formed as a single common pad. This decreases the cost of the solution, since only 3 pads are necessary instead of four.
The first pad and the third pad can be formed as a single common pad. This decreases the cost of the solution, since only 3 pads are necessary instead of four.
Each pad can be a trapezoidal shape with two long sides and two short sides, the long sides extending substantially radially from the center portion of the top surface of the scale. This shape turns out to be particularly beneficial to take into account any type of users, i.e. tall individuals having long feet and small individuals having small feet, because the respective placement of the feet is such that the position of the front portion of the foot and the rear portion of the foot are optimal for a good contact with the pads at the best location below the foot.

### BRIEF DESCRIPTION OF THE DRAWINGS

Other features and advantages of the invention appear from the following detailed description of one of its embodiments, given by way of non-limiting example, and with reference to the accompanying drawings, in which:
- Figure 1 illustrates a body of a user standing on a weighing scale according to the invention,
- Figure 2 is a closer side view showing one of the foot of the user,
- Figure 3 is a top schematic view of the weighing scale, the right half illustrating a first embodiment, and the left half illustrating an alternative embodiment,
- Figure 4 is a time chart showing various signals relating to the heart activity,
- Figure 5 illustrates an exemplary functional diagram of the scale
- Figure 6 illustrates a system comprising the scale for managing users profiles.

### DETAILLED DESCRIPTION OF THE DISCLOSURE

In the figures, the same references denote identical or similar elements.

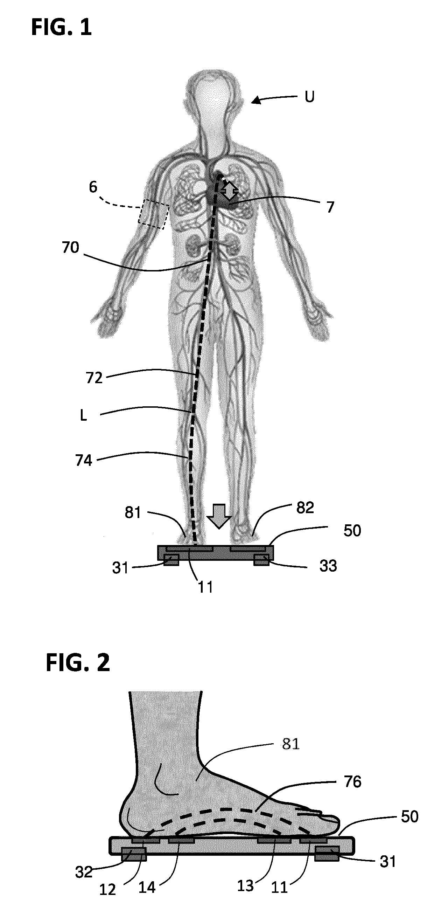

Figure 1 shows an individual **user U** standing on a weighing scale **1** (also often called 'bathroom scale'). The body of the user is shown translucent, the **heart 7** produces a pressure pulse in the arterial network causing the user's blood to circulate in arteries toward lungs, head and all other organs, blood coming back to the heart via veins.

In particular, the left ventricular contraction periodically imparts a pressure pulse in the arteries responsible for the pulsatile movement blood in the arteries from the heart towards the other organs. More particularly, the pressure pulse and the blood move toward the **feet 81,82** via the descending aorta **70,** the femoral artery **72,** and the tibial artery **74.**

Of particular interest for the following description, at each ventricular contraction, the pulsatile movement of blood in the arteries is accompanied by a recoil effect of the body which reflects into a small change in weight sensed by the weight sensors of the scale.

Besides, each ventricular contraction induces pressure pulse through the aorta **70** and the leg arteries **72,74** down to the feet. This pressure pulse sets in motion the blood in the arteries. When this pressure pulse arrives at the feet, the resulting change of volume of the blood in the feet arteries can be measured by the method known as impedancemetry.

The pressure pulse travels for a certain time from the heart to the feet. This travel time is somehow representative of the health state of the circulatory system of the user. More precisely, this travel time is representative of the arterial stiffness of the circulatory system of the user. The velocity of the blood pressure pulse is usually comprised between 5 m/s and 15 m/s.

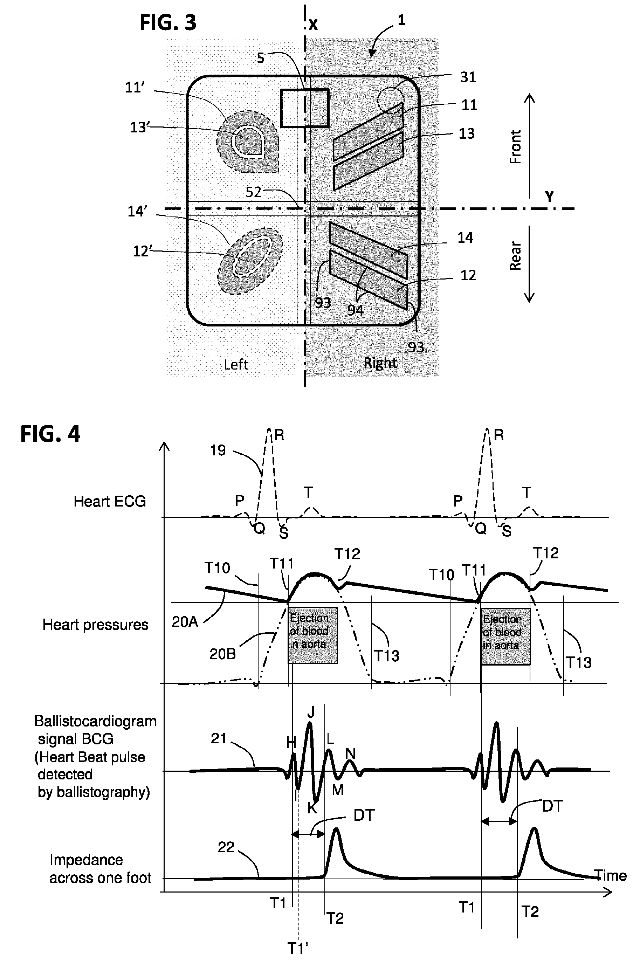

As shown in Figures 2, 3, 4 and 5, the scale has a controller **4,** a battery **8** and a display **5,** and comprises as known per se weight sensing element(s) **31,32,33,34,** for example four strain gauges as described in WO2014106716 the content of which is incorporated here by reference. The main function of the scale **1** is to determine the weight of a person standing on the scale. Also, the small variations over time of the sensed weight can be used to extract signals representative of certain physiological activity of the human body, in particular regarding the heart, this technique is called **ballistocardiography.** In particular, the heart beat activity reflects in small variations over time of the sensed weight, which are reflected in a ballistocardiogram (in short **'BCG'**), as shown at ref **21** in Fig 4. The extraction can be performed as explained with a comprehensive manner in WO2014106716. Shortly, the four strain gauges are arranged two by two, in two Wheastone bridges **35,36,** either in a right-left logic or in a front-rear logic.

Each Wheastone bridge ouputs a respective signal **78,79,** forwarded to the controller 4, where they enter into a sum-device and then further into an analog-to-digital converter or first into analog-to-digital converters and then further into a sum-device (not shown) to calculate the weight **W** therefrom, as known per se.

One solution, among others, to work out ballistogram signals, is to pick-up signals at the outputs of the Wheastone bridges **35,36,** enter them into band pass filters **37,38,** sum the resulting signals **dW1**, **dW2** in a sum-device **39** and input such signal **21** into the controller **4.**

Of course, it is possible, conversely, to perform summing before filtering, in order to issue a ballistogram signal **21.** This is referred to as step **/a**/ of the disclosed method.

It is not excluded to directly convert analog signals output by the Wheastone bridges **35,36** and perform all the subsequent treatments with digital operations within the controller. Band pass filters **37,38** can have the following cut off frequencies [0.5 Hz - 25 Hz] which discards continuous and low frequency components and also eliminates noise.

Further, the scale comprises, on its top surface **50,** at the right side of the scale, four **conductive pads 11-14,** intended to come in contact with the right foot of a person standing on the scale. As drawn, right and left sides of the scale are separated by a medial sagittal axis **X,** and front and rear portions of the scale are separated by a medial transverse axis **Y.**

The user can stand preferably barefoot on the scale; however, even if the user bears socks, it does not prevent the disclosed method to operate properly.

An electrical current is injected between a first pad **11** and a second pad **12,** and this current flows through the foot along path **76** inside the foot. This current is not harmful and not dangerous, it is limited in amps to less than **0,5 mA.**

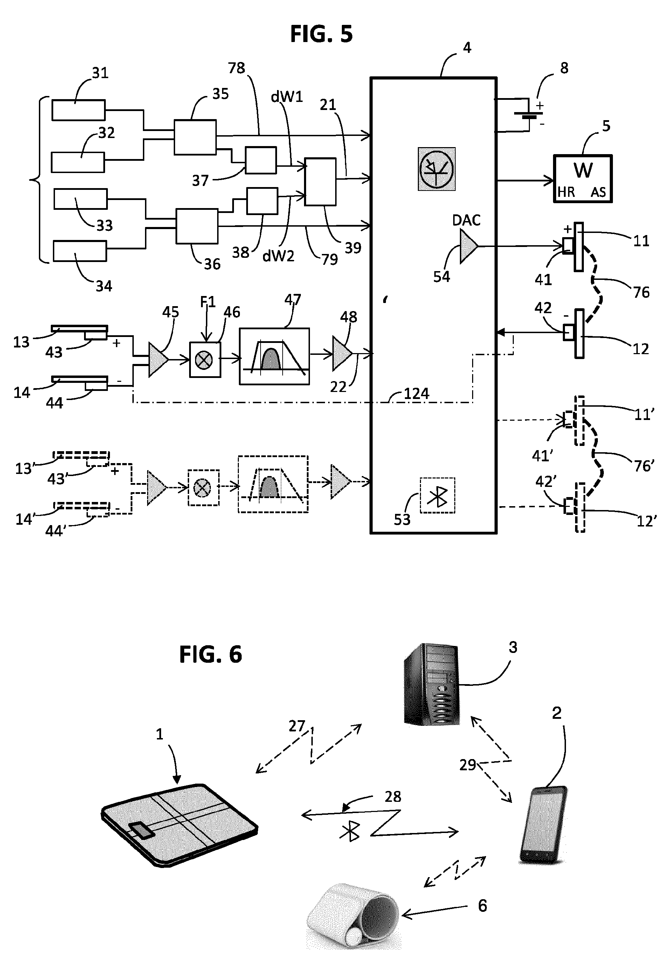

This current can be generated by a current source or a voltage source. The first conductive pad **11** is coupled to a first electrode **41** which is coupled to a current output of the scale, controlled by a current or voltage control signal of the controller **4** (via for instance a Digital Analog Converter **54,** or another method (not shown), and adequate signal conditioning (not shown), cf. Fig 5). The first conductive pad **11** is located at a front portion of the top surface of the scale and is conventionally the place where current is entered into the foot of the user ('+' terminal).

The second pad **12** is coupled with a second electrode **42** which is coupled to a current input (also called 'current return') of the scale reference. The second pad **12** is located at a rear portion of the top surface of the scale and is conventionally the place where current comes out of the foot of the user ('-' terminal).

Advantageously the injected current is a sine alternating current. The applied frequency **F1** is in the range [10kHz-200kHz], preferably about 50kHz, such that the current injection is not harmful to the user and unnoticed by him. Preferably the injected current has a predefined fixed frequency F1 and a steady amplitude, and is generated by a current source or a voltage source.

Blood arriving in the foot produces a modulation (at the frequency of the heart rate) of the impedance. The amplitude of the modulation is rather small, it accounts for about 1/1000 of the impedance of the body (foot to foot).

Simultaneously with the current injection, a resulting voltage is measured across a third pad 13 and a fourth pad 14. Since the voltage is modulated at the same frequency as per the injected current, demodulation is required to extract the baseband frequency voltage exhibiting only the low frequency modulation induced by the blood volume variation, as detailed below.

The third pad **13** is coupled with a third electrode **43** which is coupled to a first voltage input of the controller. The third pad **13** is located at the front portion of the top surface of the scale, close to the first pad.

The fourth pad **14** is coupled with a fourth electrode **44** which is coupled to a second voltage input of the controller. Advantageously, a differential measuring technique is carried out.

The fourth pad **14** is located at the rear portion of the top surface of the scale close to the second pad.

As illustrated, the third pad 13 and the fourth pad 14 are interposed between the first pad 11 and the second pad 12; in other words, measured voltage is picked up inside the current injection area in the foot.

In an alternative embodiment, the first pad 11 and the second pad 12 are side by side at the front portion of the plate, and the third pad 13 and the fourth pad 14 are side by side at the rear portion of the plate. In the "parallel" configuration, the electrodes are such that the current is injected and picked up on the interior of the foot, and the voltage is measured on the exterior of the foot. Alternately, the current injection can be on the exterior of the foot and voltage measurement can be on the interior. In the "cross" configuration, the electrodes for the current injection are along one diagonal, and the electrodes for voltage measurement are along the second diagonal.

In an alternative reversed embodiment, the first pad **11** and the third pad **13** could be arranged at the rear portion (instead of front portion), and the second pad **12** and the fourth pad **14** could be are arranged at the front portion (instead of rear portion).

More precisely, as already mentioned, the periodic heart beat induces a small periodic blood volume variation in the foot; and since the blood volume variations in the foot results in corresponding electrical impedance, impedance variations are representative of the blood volume variations which are resulting in turn from the blood flow pulse arriving at the foot from the heart. This is also known as "impedance plethysmography" (**'IPG'** in short).

In other words, the scale controller **4** acquires impedance plethysmography signals across the foot of the user resulting from a **blood flow pulse** at the foot, in particular a variation of the impedance, resulting from a corresponding variation of the blood volume at the foot.

Therefore the IPG signal **22** will be the result of a demodulation of the voltage measured between pads 13 and 14, such demodulation being performed by a dedicated hardware block upfront the controller.

More precisely, with reference to Fig 5, circuit **45** is an amplifier which amplifies the voltage difference between electrodes **44** and **43.** Circuit **46** is an amplitude demodulator, to issue a baseband frequency signal. Circuit **47** is a band pass filter and circuit **48** is another amplifier to result in a ready-to-use impedance plethysmography signal **22.**

The thus demodulated and filtered analog voltage is digitally handled by the controller 4.

This is referred to as step **/b/** of the disclosed method.

The stages of the electronic chain can be exchanged. For instance demodulation can be done before amplification.

Advantageously, the current between the pads 13 and 14 can be measured in addition to the voltage in order to improve the accuracy of the impedancemetry measurement by removing the possible perturbation caused by variations of the contact impedance due to the person's imperceptible motion.

It is to be noted that the current input 12 and the second voltage input 14 are distinct and separate, as illustrated, to enhance accuracy and signal decoupling. However, in a variant embodiment, the current input 12 and the second voltage input 14 can be electrical-wise common (chain-dotted line **124** at figure 5). In another variant embodiment, not shown, the second and fourth pads **12,14** are formed as a single pad, such that only three conductive pads (instead of 4) are sufficient to measure the impedance of the foot. In another variant embodiment, not shown, current input 11 and the second voltage input 13 can be electrical-wise common. In another variant embodiment, not shown, the first and third pads **11, 13** are formed as a single pad. In another variant, not shown, the fourth pad 14 and electrode 43 are removed and the voltage is measured between pad 13 and the ground of the electrical circuit.

The impedance plethysmography signal 22 resulting from the above described signal conditioning is shown at Fig 4, with other signals.

Signal **19** shows an indicative heart electrocardiogram (ECG) reflecting the heart electrical activity, as known per se.

Signals **20A** and **20B** show superposed respectively the ventricular (20B) and aortic (20A) pressures during cardiac cycles. The mechanical contraction of the heart causes the rise of the ventricular pressure. **T10** denotes the closing of the mitral valve, inducing the beginning of the pressure rise in ventricle (isovolumic contraction); at the instant **T11**, when the ventricular pressure 20B equals the diastolic pressure in the aorta, the aortic valve opens and blood is ejected from the ventricle into the aorta, this phase lasts until the instant **T12** when the ventricular pressure 20B becomes lower than the aortic pressure, with the closure of the aortic valve. **T13** denotes the return of the ventricle to an idle state.

Besides, BCG signal **21** shows the corresponding ballistocardiogram (responsive to heart beat), which exhibits a periodic occurrence of a pulse-like wave having negative apexes **I,K,M** and positive apexes **H,J,L,N.** Instant **T1** is defined to be the first positive apex **H.** Instant **T1'** is defined to be the first negative apex **I**. Either **T1** and **T1'** can be used to estimates of the opening of the aortic valve at **T11**. Alternately, other markers of the BCG could be used to estimate the opening of the aortic valve at **T11,** for instance the instant of the maximum of the time derivative of the BCG between H and I.

As discussed above, the impedance plethysmography resulting **signal** 22 is responsive to an increase of the blood volume. Instant **T2** is defined to be the first detected significant rise in the signal.

The time difference **T2-T1** is related to the pulse transit time (PTT) of the pressure pulse from the heart to the foot. We note **DT=T2-T1,** and this time delay calculation is referred to as step **/c/** of the disclosed method.

**DT** can be the averaged result of three or more consecutive calculations, for more accuracy and/or reliability.

**DT** can typically be comprised between 50ms and 300ms, generally between 80ms and 200ms. For a normal young individual, the arteries are flexible, and the time delay **DT** is rather long, typically 120ms or more depending on his height. For a normal old individual, the arteries are more rigid, and the time delay DT is shorter, typically 110ms or less depending on his height. Of course, these values are indicative only. Certain young individuals may have time delays shorter than 120ms, as well as certain old individuals may have time delays longer than 110ms.

On the display **5,** the user can read the weight **W,** the heart rate **HR** and a value of arterial stiffness **AS.** The arterial stiffness **AS** stands for the flexibility of arteries wall tissues. HR can be determined from the BCG signal 21 and/or from IPG signal 22.

One way to express Arterial Stiffness **AS** is to use the **pulse wave velocity (PWV)** of the pressure pulse. It is calculated as **PWV = f (L/DT)** with f being a linking function.

The path length **L** from the heart to the foot is calculated with a function of the height of the user. **DT,** as explained above is related to the pulse transit time of the blood pressure pulse.

**DT** depends on the blood pressure and the stiffness of arteries.

The function f is a parametrization which depends on the age and gender of the user, and also depends in a lesser extent on a blood pressure type (normal, hypertensed, ..) and the height/weight of the user and optionally also the blood pressure type.

**PWV** can therefore be expressed in m/s. The PWV of the user can be compared to a normal range given the age and gender of the user.

Another way to express Arterial Stiffness is as an arterial equivalent age, or an arterial range of age, reflecting the state of the arteries compared to a normal state given the chronological age and gender of the user. Therefore, the display 5 can write for example an interval **[23 y/o** - **26 y/o].**

A value for the arterial stiffness can be given either at each measurement, or can be profitably averaged over several subsequent measurements to smooth out daily variations.

A arterial stiffness value found outside the expected range for an individual may denote some cardiovascular problem, an atherosclerosis or atheromatosis.

As illustrated in figure 6, the scale **1** is used preferably in a system comprising a smartphone **2** or the like and a remote server **3** (or cloud service).

The scale **1** and the smartphone **2** are able to be in communication through a wireless short-range communication link **28,** preferably Bluetooth™ **53** interface. However, instead of Bluetooth™, any wireless remote short-range communication link can be used.

As known per se, the smartphone **2** is able to be in communication through cellular wireless network **29** with generally speaking internet, and particularly the remote server **3** (or the cloud service). It is not excluded to have a direct link **27** from scale **1** to the remote server **3** (or cloud service).

Each individual which may use the scale can be defined at least by a user profile which comprises the height, the age and the gender of the individual. This data can be entered via the graphic tactile interface of the smartphone, and can be stored in the server 3.

Also, the scale **1** can recognize automatically which user is currently standing on it, thanks to weight expected intervals, as taught in US8639226.

The height, the age and the gender of the individual are used to adjust the interpretation of the value of DT (or PWV) with regard to normally expected values, i.e. min-max normal interval for a particular type of individual.

The height, the age and the gender of each known individual are sent from the smartphone down to the scale, for example, at the first use.

There may be provided abacus or regression curves in the server 3 to which the user measured values are compared. There may be provided individual storage with past measurements which constitutes a personal history data, stored either in the smartphone and/or in the server 3.

The system can also comprise a blood **pressure monitor device 6.** From time to time, the user measures its blood pressure. The resulting blood pressure data is sent to the smartphone 2 and can be used to adjust the arterial equivalent age from the values of PWV.

Regarding size and shape of conductive pads **11-14,** in a preferred embodiment illustrated on the right side at figure 3, each pad can be a trapezoidal shape with two long sides (segments) **94** and two short sides **93,** the long sides extending substantially radially from the center portion **52** (where axis X and Y cross) of the top surface **50** of the scale.

On Figure 3 and Figure 5, there are shown in dotted line additional conductive pads **11',12',13',14',** which can be seen functionally as a duplicate of the already commented pads at the other side of the scale. Similarly, additional electrodes **41'-44'** are used to connect the additional conductive pads **11'-14'** to the internal electrical circuits of the scale 1.

There are provided eight conductive pads in the shown example. However, there may be provided 6 pads (three pads per side) with the common ground configuration explained above.

With this duplicate (right+left) configuration, steps /a/ to /d/ of the method can be performed both for the left foot and for the right foot, and a resulting average value is outputted (mean value on left and right feet). Also an additional notice is given if the left and right values differ of more than a predetermined value, which indicates a vascular problem in one leg.

However, it should be understood that impedance plethysmography signal 22 can be obtained from a single side (on only one foot).

It is noted that the overall average impedance Z from foot to foot can be performed using the above mentioned items, especially a subset of the electrodes used to measure the impedance plethysmogram in one foot. One solution is to provide electronic switches that allow to inject a current from one of the front right pads 11, 13 to one of the front left pads 11', 13' and to measure the voltage between one of the rear right pads 12, 14 (or the corresponding unique electrode if they are confounded) and one of the rear left pads 12',14' (or the corresponding unique electrode if they are confounded). The roles of the injecting and measuring pairs of electrodes can be interchanged.

Therefore, it is possible to combine the conventional body fat percentage measurement with the single foot blood pulse time analysis.

As illustrated on the left part of figure 3, the conductive pads can have the shape of waterdrop, an ovoid shape, etc... Further, one conductive pad can be encircled by another. An interlaced configuration is not excluded.

Advantageously, the value of the arterial stiffness is compared to a previous value of the arterial stiffness of the same user (accessible in user personal history data at server 3), so that a change over time in the user's cardiovascular system can be found.

## Claims

1. **Method** to determine the arterial stiffness of an individual user (U) standing on a personal electronic scale (1) having a top surface with at least three conductive pads (11-13) on one of the left or right side of the scale, the method comprising :
**/a/-** acquiring weight variations, and extracting therefrom a ballistocardiogram (21) of the user's heart beat,
**/b/-** acquiring impedance plethysmography signals across one of the feet of the user, and extracting therefrom a blood pulse signal (22) at the foot,
**/c/**-calculating a time delay (DT) between the heart beat and the blood pulse signal arriving at the foot,
**/d/** deducing therefrom a value of the arterial stiffness of the user.

2. The method of claim 1, wherein step **/b/** comprises:
**/b1/-** injecting into the foot, a current between a first pad (11) and a second pad (12)
**/b2/-** measuring resulting voltage across a third pad (13) and a fourth pad (14),

3. The method of claim 2, wherein at step **/b1/** the injected current is a sine alternating current.

4. The method of claim 1, wherein at step **/d/** the deduction of a value of the arterial stiffness is made according to a profile of the user, said profile including the height, the age, the gender and the blood pressure of the user.

5. The method of claim 1, wherein at step **/d/** the value of the arterial stiffness is compared to a previous value of the arterial stiffness of the same user.

6. The method of claim 1, in which the time delay (DT) is calculated as an average over at least 3 heart beats.

7. The method of claim 1, wherein there are provided at least three conductive pads (11-13) on the left side of the scale and at least three conductive pads (11-13) on the right side of the scale, and wherein steps **/a/** to **/d/** are performed for the left foot and for the right foot, and a resulting average value is outputted together with a notice if the left and right values differ of more than a predetermined value.

8. An electronic scale (1) having at least three conductive pads (11-13) on one of the left or right side of a top surface (50) of the scale, wherein a first and a second pads are used to circulate a current into one foot of a user, and wherein a third and a fourth pads are used to measure the resulting voltage current across the same foot.

9. The electronic scale of claim 8, wherein the first pad (11) and the third pad (13) are arranged on the right side front portion of the top surface of the scale, and the second pad (12) and the fourth pad (14) are arranged on the right side rear portion of the top surface of the scale.

10. The method of claim 8, wherein the fourth pad (14) and the second pad (12) can be formed as a single common pad.

11. The method of claim 8, wherein each pad can be a trapezoidal shape with two long sides and two short sides, the long sides extending substantially radially from the center portion (52) of the top surface (50) of the scale.
